# EUROPEAN PATENT SPECIFICATION

(11) **EP 1 733 764 B1**
(45) Date of publication and mention of the grant of the patent: **25.03.2015**
(21) Application number: 06076698.7
(22) Date of filing: 28.03.2002
(51) Int. Cl.: A62D 1/00, C07C 43/00, C07C 43/12

(54) **Fire and explosion suppression agent**
Mittel zum Feuerlöschen und zur Explosionsunterdrückung
Agent de suppression d'explosion et d'incendie

(30) Priority: 29.03.2001 GB 0107886; 27.07.2001 GB 0118374
(43) Date of publication of application: 20.12.2006
(62) Divisional of application: 02708510.9
(73) Proprietor: Kidde IP Holdings Limited, Slough, Berkshire SL3 0HB (GB)
(72) Inventor: Grigg, Julian, Burnham Berkshire SL1 6HP (GB)
(74) Representative: Chiva, Andrew Peter

(56) References cited:
- EP-A1- 0 562 756
- WO-A2-01/05468
- US-A- 3 666 864
- US-A- 3 897 502
- US-A- 5 141 654
- HUDLICKY T; DUAN C; REED JW; YAN F; HUDLICKY M; ENDOMA M A; EGER E I: "Practical preparation of potentially anesthetic fluorinated ethyl methyl ethers by means of bromine trifluoride and other methods" JOURNAL OF FLUORINE CHEMISTRY, vol. 102, 2000, pages 363-367, XP002215665

## Description

### BACKGROUND OF THE INVENTION

### 1. Field of the Invention

The invention relates to fire and explosion suppression. Embodiments of the invention, to be described below by way of example only, use liquid suppressants in mist form. The suppressants used are intended to deal with the problems of ozone depletion and global warming.

### 2. Description of the Related Art

It is known (e.g. from GB-A-2 265 309) to extinguish fires or explosions by discharging a liquid chemical fire extinguishing substance in mist form in suspension in an inert gas.

It is also known from WO-A-015468 to discharge a chemical fire extinguishing substance in liquid form by means of an inert gas.

### BRIEF SUMMARY OF THE INVENTION

According to the invention, there is provided a fire or explosion suppression agent, having two suppressant parts, one comprising an explosion suppressing chemical substance which is substantially liquid at normal temperatures and pressures and the other comprising a fire or explosion suppressing inert gas; the chemical substance being dispersed as a suspension in the inert gas, the chemical substance when so disposed having low environmental impact, with a short atmospheric lifetime of less than 30 days; the chemical substance comprising one or more chemicals with the structure **Z-R-X-Y,** where the monovalent radical **Z** is a halogen atom taken from the group fluorine **(-F)** or bromine **(-Br);** where the divalent radical **R** is a perfluoro- or polyfluoro-alkylidene group of formula **-CₙHₚF₂ₙ₋ₚ-**, with **n** in the range 1-6, and **p** in the range 0-4; where the divalent radical **X** is carbonyl **(-CO-);** and where the monovalent radical **Y** is selected from the group hydrogen **(-H),** bromine **(-Br),** alkyl of formula **-CₘH₂ₘ₊₁** with m in the range 1-4, or perfluoroalkyl of formula **-CₘF₂ₘ₊₁** with **m** in the range 1-4, or polyfluoroalkyl of formula **-CₘHₖF₂ₘ₊₁₋ₖ** with **m** in the range 1-4 and **k** in the range 1-2m; the agent including nothing else having any significant environmental impact and which has an atmospheric lifetime longer than 30 days.

According to the invention, there is also provided a method of suppressing a fire or explosion, in which a fire or explosion suppressing chemical substance which is in liquid form or substantially so at normal temperatures and pressures is dispersed as a suspension in a fire or explosion suppressing inert gas and discharged with the gas into an area to be protected; the chemical substance being dispersed as a suspension in the inert gas, the chemical substance when so disposed having low environmental impact, with a short atmospheric lifetime of less than 30 days; the chemical substance comprising one or more chemicals of the structure **Z-R-X-Y,** where the monovalent radical **Z** is a halogen atom taken from the group fluorine **(-F)** or bromine **(-Br);** where the divalent radical **R** is a perfluoro- or polyfluoro-alkylidene group of formula **-CₙHₚF₂ₙ₋ₚ-,** with **n** in the range 1-6 and **p** in the range 0-4; where the divalent radical **X** is carbonyl **(-CO-)** and where the monovalent radical **Y** is selected from the group hydrogen **(-H),** bromine **(-Br),** alkyl of formula **-CₘH₂ₘ₊₁** with **m** in the range 1-4, or perfluoroalkyl of formula **-CₘF₂ₘ₊₁** with **m** in the range 1-4, or polyfluoroalkyl of formula **-CₘHₖF₂ₘ₊₁₋ₖ** with **m** in the range 1-4 and **k** in the range 1-2m; the agent including nothing else having any significant environmental impact and which has an atmospheric lifetime longer than 30 days.

According to the invention, there is further provided a fire or explosion suppressant system, comprising a source (30;5) of a fire or explosion suppressing chemical substance which is in liquid form or substantially so at normal temperatures and pressures, and a source (32;14) of a pressurised fire or explosion suppressing inert gas, means (34;6) for dispersing the chemical substance as a suspension in the pressurised gas, and discharge means (44;26,29) for discharging the so-dispersed chemical substance and the pressurised gas into an area to be protected; the chemical substance being dispersed as a suspension in the inert gas, the chemical substance when so disposed having low environmental impact, with a short atmospheric lifetime of less than 30 days; the chemical substance comprising one or more chemicals with the structure **Z-R-X-Y,** where the monovalent radical **Z** is a halogen atom taken from the group fluorine **(-F)** or bromine **(-Br);** where the divalent radical **R** is a perfluoro- or polyfluoro-alkylidene group of forrnula - **CₙHₚF₂ₙ₋p**- with n in the range 1 - 6 and **p** in the range 0-4; where the divalent radical **X** is carbonyl **(-CO-);** and where the monovalent radical **Y** is selected from the group hydrogen **(-H),** bromine **(-Br),** alkyl of formula **-CₘH₂ₘ₊₁** with **m** in the range 1- 4, or perfluoroalkyl of formula - **CₘF₂ₘ₊₁** with **m** in the range 1- 4, or polyfluoroalkyl of formula -**CₘHₖF₂ₘ₊₁₋ₖ** with **m** in the range 1-4 and **k** in the range 1-2m; the agent including nothing else having any significant environmental impact and which has an atmospheric lifetime longer than 30 days.

### BRIEF DESCRIPTION OF THE DRAWINGS

Fire and explosion suppression systems and methods according to the invention, employing mists, will now be described by way of example only, with reference to the accompanying diagrammatic drawings in which:
Figure 1 is a schematic diagram of one of the systems; and
Figure 2 is a schematic diagram of another of the systems.

### DETAILED DESCRIPTION OF EMBODIMENTS OF THE INVENTION

Halons (Halons 1301 and 1211) have been used in the past as fire and explosion extinguishants and suppressants. Their physical and toxicological properties and extinguishing efficiency made them ideal for total flooding and streaming applications. They are efficient extinguishing agents because they contain bromine atoms which terminate the radical chain reactions that propagate combustion by catalytic reactions. These same bromine atoms are now known to catalytically remove ozone in the stratosphere. Therefore, Halons have an ozone depletion potential (ODP) and their production was ceased at the end of 1993. Since then, many alternative fire suppressants have reached the market place. Currently, hydrofluorocarbons dominate the industrial and commercial markets. However, aerospace, military and specialised uses are still dependent upon recycled Halon for space and weight efficiency reasons; the current Halon replacement agents are not as efficient as Halons for fire extinguishing.

Another factor that indicates the environmental impact of an extinguishing agent is its global warming potential (GWP). This parameter is related to the atmospheric lifetime of a molecule and is becoming increasingly important and will continue to do so in the future. This is especially true following the Kyoto Protocol and greenhouse gas emission targets. Hydrofluorocarbons have an ODP of zero but they have material atmospheric lifetimes. As a result, their use is likely to be subject to restriction in the future.

Extinguishing agents with short atmospheric lifetimes are desirable.

There are several basic mechanisms for the breakdown of organic molecules released into the atmosphere :-
1. **Reaction with ·OH radicals:** this is the principal tropospheric degradation mechanism for most organic molecules. The most common reaction is that of hydrogen atom abstraction.

   X-H + ·OH → ·X + H₂O (slow)

   ·X → → final products (fast)

   The rate of the whole process is controlled by the rate of the first reaction, the hydrogen abstraction reaction. The radical ·X then breaks down very rapidly to the final products such as CO₂, H₂O, HF, HBr etc. which are washed out of the atmosphere in rain. Clearly the molecule must possess an abstractable hydrogen atom for this reaction to occur. There is also another possibility, namely addition of the ·OH radical to a double bond, *e.g.* 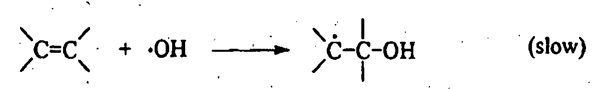 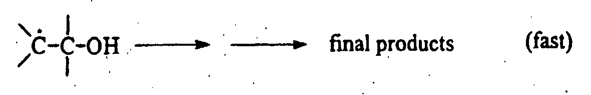
2. **Hydrolysis:** provided that the molecule contains hydrolytically unstable bonds, the reaction of a molecule with water generates water soluble molecules which are then rapidly washed out of the atmosphere in rain.
3. **Photolysis:** providing the molecule contains a UV-absorbing chromophore, such as a double bond, C=C or C=O, then degradation in the troposphere may occur readily.
4. **Reaction with O₃ and NO₃:** these two species contribute only a very minor part of the tropospheric degradation mechanisms in comparison with the -OH reaction route.

It is therefore possible to limit the atmospheric lifetime of gaseous extinguishing molecules by the introduction of substituents into the molecule that will yield a high rate of reaction with ·OH radicals or substituents that will cause the molecule to decompose by photolysis in the troposphere. These molecules are said to be tropodegradable. Such substituents include the ether group (-O-), a carbonyl group (-CO-) and an alkene group (-C=C-). This strategy allows molecules that contain bromine to be used as extinguishing agents because the short atmospheric lifetimes mean that the agents do not get into the stratosphere where ozone depletion is a problem. However, the inclusion of these groups increases the molecular weight of the agent molecule. This increases the boiling point and gives the corresponding lowering of the vapour pressure. As a result, the tropodegradable extinguishing agents are likely to be liquids at room temperature and pressure.

Because total flooding applications require three dimensional distribution such as occurs with a gaseous agent, liquid extinguishing agents have not been considered in the past. Indeed, to a person skilled in the art of fire protection science, they would be dismissed from consideration because of these volatility issues.

Thus at present, suppressants that are essentially liquid at normal temperatures and pressures can be deployed for extinguishing fires using, for example, appliances such as hand-held fire extinguishers which deploy the suppressants in their normal form. They may be satisfactory in such applications but, because they are deployed in liquid form (e.g. as a liquid stream), they must be more or less directed at the fire for maximum effectiveness. They cannot be deployed in this way as a total flooding agent - that is, such as in gaseous or liquid form from which they will expand to fill a space in which a fire or explosion may exist or in which a fire or explosion is to be prevented. In many applications, such a total flooding capability is important in order to ensure that a specified space or volume (such as a room or the interior of a vehicle or a volume within an aircraft) can be more or less filled with the suppressant.

The systems and methods to be described are therefore essentially concerned with particular chemical suppressants which are in liquid form, or substantially so, at normal temperatures and pressures, and enable such suppressants, in spite of their liquid form, to be deployed as total flooding agents.

The chemical fire suppressants to be described have low environmental impact, with a short atmospheric lifetime of less than 30 days. More specifically, they comprise one or more chemicals with the structure **Z-R-X-Y,** where the monovalent radical **Z** is a halogen atom taken from the group fluorine **(-F),** or bromine **(-Br);** where the divalent radical **R** is a perfluoro- or polyfluoro-alkylidene group of formula **-CₙHₚF₂ₙ₋ₚ**- with **n** in the range 1 - 6 and **p** in the range 0 - 4; where the divalent radical **X** is carbonyl **(-CO-);** where the monovalent radical **Y** is selected from the group hydrogen **(-H),** bromine **(-Br),** alkyl of formula -**CₘH₂ₘ₊₁** with **m** in the range **1-4,** or perfluoroalkyl of formula -**CₘF₂ₘ₊₁** with **m** in the range **1-4,** or polyfluoroalkyl of formula **-CₘHₖF₂ₘ₊₁₋ₖ** with **m** in the range **1-4** and **k** in the range **1-2m;** and where, optionally, the radicals **R** and **Y** may be linked (by a C-C bond) such as to form a 4-, 5-, or 6- membered ring.

Preferably, the groups **Z,X** and **Y** are so selected that the total number of bromine atoms in the molecule does not exceed one.

Preferably, the groups **R** and **Y are** selected such that **n** + **m** lies in the range 1 - 6 with the further proviso that **n** - **m** must be at least 1.

Preferably, the groups **R,X,** and **Y** are chosen so that the total number of carbon atoms in the molecule is in the range 3 - 8, and very preferably in the range 3 - 6.

Preferably, the molecular weight of the molecule lies in the range 150 - 400, and very preferably in the range 150 - 350.

Preferably, the groups **R,X** and **Y** are chosen so the weight % of halogen (fluorine and bromine) in the molecule lies in the range 70 - 90%, and very preferably in the range 70-80%.

A specific example of a suitable suppressant is dodecafluoro-2-methylpentan-3-one which has the formula CF₃CF₂C(O)CF(CF₃)₂. This compound has a molecular weight of 316, it contains 72% halogen and has a boiling point at 1 atmosphere of 48°C. The n-Heptane cupburner extinguishing concentration of the compound in volume % is 4.5 ± 0.1. The compound is tropodegradable due to photolysis of the CO group and has an estimated atmospheric lifetime of 5 days.

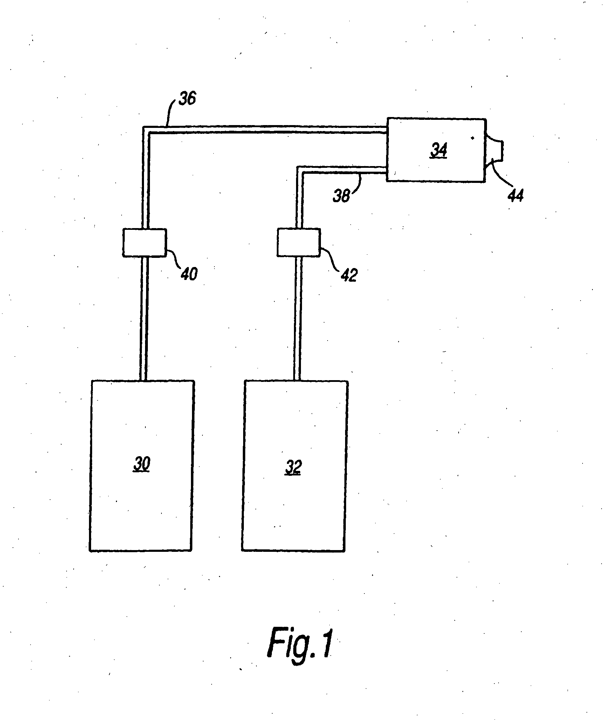

Figure 1 shows how such a liquid suppressant may be deployed in mist form. As shown in Figure 1, the liquid suppressant is stored under pressure in a suitable vessel 30. An inert gas, typically nitrogen, is stored under pressure in a second vessel 32. The vessels 30 and 32 are respectively connected to an output unit 34 by pipes 36 and 38 and control valves 40 and 42. When the control valves 40 and 42 are opened, the liquid suppressant and the inert gas are fed under pressure to the output unit 34. The output unit 34 comprises a hollow chamber into which the liquid suppressant and the inert gas are discharged. Within the mixing chamber, the gas and the liquid physically interact and the gas causes the suppressant to be formed into a mist made up of droplets of small size, preferably in the range of between 5 and 60 micrometres. The mist is produced partly by a shearing action of the gas on the liquid suppressant. Within the unit 34, the liquid suppressant may enter in a direction substantially parallel to the direction of the gas. Instead, it can enter substantially at right angles to the gas and the shearing action will be greater. Another possibility is for the liquid suppressant to enter in a direction opposite to that of the gas, and the shearing action may be greater still. After the liquid agent and inert gas have been mixed, vapour from the liquid agent will also be formed. The resultant vapour and mist of the liquid suppressant together with the inert gas, which carries them, exits through a nozzle 44 into the volume or area to be protected.

The combination of vapour and liquid mist dispersed in the inert gas now forms a suppression agent having some of the characteristics of a gaseous suppressant. In particular, because the vapour and mist are being carried by the inert gas they can permeate and expand into all or most parts of the space or volume to be protected and thus provide a total flooding capability. The suppressant agent of course includes nothing else having any significant environmental impact and which has an atmospheric lifetime longer than 30 days.

The output unit 34 may be arranged to supply more than one nozzle 44. More particularly, it may supply a pipework array with multiple nozzles.

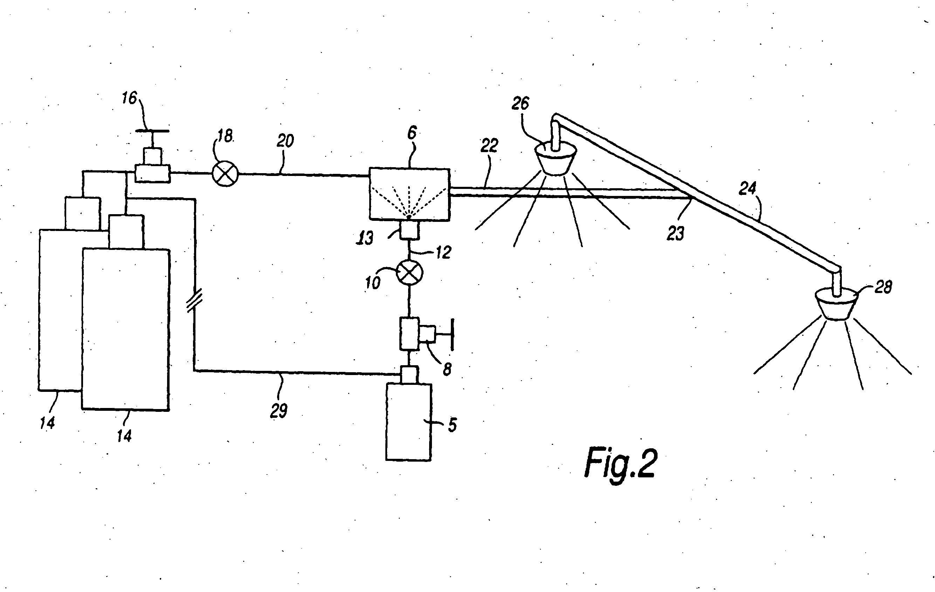

Figure 2 shows another system for deploying such a liquid suppressant in mist form and carried by an inert gas, the system having similarities with the form disclosed in our copending United Kingdom patent application No. 0123146.3 (Serial No. ).

In Figure 2, a vessel 5 stores the liquid suppressant under pressure. The vessel 5 is connected to an input of a mixing unit 6 via a pressure regulator 8, a flow regulator 10, a pipe 12, and a nozzle 13.

The system also includes vessels 14 storing an inert gas such as nitrogen which has an outlet connected via a pressure regulator 16, a flow regulator 18 and a pipe 20 to another input of the mixing unit 6. The mixing unit 6 has an outlet pipe 22 which connects with the distribution pipe 24 terminating in spreader or distribution heads 26, 28. The liquid suppressant in the vessel 5 may be pressurised by the gas in the vessels 14 via a pipe 29. However, it may be pressurised in some other way.

In use, the liquid suppressant from the vessel 5 is fed under pressure into the mixing unit 6 and enters the mixing unit 6 via the nozzle 13 which is arranged to convert the liquid suppressant into a mist of droplets of small size, again preferably in the range of between 5 and 60 micrometers. The mist may be produced simply by the step of forcing the liquid through the nozzle 13. Instead, the nozzle may incorporate means such as a rotary atomising disk to produce or augment the misting process.

Additionally, the mist of the liquid suppressant is mixed within the mixing chamber 6 with inert gas and becomes disposed as a suspension within the gas. Vapour is also formed as the liquid droplets evaporate by virtue of their high surface area to volume ratio.

The mist and vapour carried by the inert gas exit the mixing chamber 6 along the outlet pipe 22 to a T-junction 23 and thence along the distribution pipe 24, and exit from the spreaders 26, 28 into the volume to be protected.

In the system of Figure 2, it is an important feature that the mixing unit 6 in which the mist is produced is separate from and distanced from the outlets or spreaders 26, 28. The mist and vapour exiting the mixing unit 6 moves at high velocity and is entrained by and within the high pressure gas. The resultant turbulence in the pipe 22 helps to reduce the size of the droplets in the mist and form vapour. The already-formed high velocity mist and vapour exit the spreaders as a two-phase mixture which consists of the inert gas carrying fine droplets and vapour of the liquid chemical extinguishant. The gas continues to expand, on exiting the spreaders 26, 28, producing an even mixture - which thus acts again as a total flooding agent.

The presence of the inert gas in the discharged mist increases the efficiency of the extinguishing and suppression action because the inert gas is a suppressant in its own right.

The systems described above with reference to Figures 1 and 2 have used nitrogen as the inert gas. Other suitable gases are argon, helium, neon and carbon dioxide or mixtures from any two or more of these gases and nitrogen. However, any other suitable gas or gas mixture may be used which is non-combustible or is effectively inert in a flame.

The extinguishants can have the advantage of being clean agents in that they leave no residue after deployment.

A mixture of the suppressants can be used.

Such systems as described with reference to Figures 1 and 2 can have fire suppressant properties similar or equivalent to those which use known total flooding extinguishing agents. They may have applications as an alternative to fixed fire suppression systems using Halons, perfluorocarbons, hydrofluorocarbons and hydrochlorofluorocarbons.

## Claims

1. A fire or explosion suppression agent, having two suppressant parts, one comprising an explosion suppressing chemical substance which is substantially liquid at normal temperatures and pressures and the other comprising a fire or explosion suppressing inert gas; the chemical substance being dispersed as a suspension in the inert gas, the chemical substance when so disposed having low environmental impact, with a short atmospheric lifetime of less than 30 days; the chemical substance comprising one or more chemicals with the structure **Z-R-X-Y,** where the monovalent radical **Z** is a halogen atom taken from the group fluorine **(-F)** or bromine **(-Br);** where the divalent radical **R** is a perfluoro- or polyfluoro-alkylidene group of formula **-CₙHₚF₂ₙ₋ₚ-,** with **n** in the range 1-6, and **p** in the range 0-4; where the divalent radical **X** is carbonyl **(-CO-);** and where the monovalent radical **Y** is selected from the group hydrogen **(-H),** bromine **(-Br),** alkyl of formula **-CₘH₂ₘ₊₁** with m in the range 1-4, or perfluoroalkyl of formula **-CₘF₂ₘ₊₁** with **m** in the range 1-4, or polyfluoroalkyl of formula **-CₘHₖF₂ₘ₊₁₋ₖ** with **m** in the range 1-4 and **k** in the range 1-2m; the agent including nothing else having any significant environmental impact and which has an atmospheric lifetime longer than 30 days.

2. A method of suppressing a fire or explosion, in which a fire or explosion suppressing chemical substance which is in liquid form or substantially so at normal temperatures and pressures is dispersed as a suspension in a fire or explosion suppressing inert gas and discharged with the gas into an area to be protected; the chemical substance being dispersed as a suspension in the inert gas, the chemical substance when so disposed having low environmental impact, with a short atmospheric lifetime of less than 30 days; the chemical substance comprising one or more chemicals of the structure **Z-R-X-Y,** where the monovalent radical **Z** is a halogen atom taken from the group fluorine (**-F**) or bromine (**-Br**); where the divalent radical **R** is a perfluoro- or polyfluoro-alkylidene group of formula **₋CₙHₚF₂ₙ₋ₚ**, with **n** in the range 1-6 and **p** in the range 0-4; where the divalent radical **X** is carbonyl (**-CO-**); and where the monovalent radical **Y** is selected from the group hydrogen (**-H**), bromine **(-Br),** alkyl of formula **-CₘH₂ₘ₊₁** with **m** in the range 1-4, or perfluoroalkyl of formula **-CₘF₂ₘ₊₁** with **m** in the range 1 - 4, or polyfluoroalkyl of formula **-CₘHₖF₂ₘ₊₁₋ₖ** with **m** in the range 1-4 and **k** in the range 1-2m; the agent including nothing else having any significant environmental impact and which has an atmospheric lifetime longer than 30 days.

3. A fire or explosion suppressant system, comprising a source (30;5) of a fire or explosion suppressing chemical substance which is in liquid form or substantially so at normal temperatures and pressures, and a source (32;14) of a pressurised fire or explosion suppressing inert gas, means (34;6) for dispersing the chemical substance as a suspension in the pressurised gas, and discharge means (44;26,29) for discharging the so-dispersed chemical substance and the pressurised gas into an area to be protected; the,chemical substance being dispersed as a suspension in the inert gas, the chemical substance when so disposed having low environmental impact, with a short atmospheric lifetime of less than 30 days; the chemical substance comprising one or more chemicals with the structure **Z-R-X-Y,** where the monovalent radical **Z** is a halogen atom taken from the group fluorine **(-F)** or bromine **(-Br)**; where the divalent radical **R** is a perfluoro- or polyfluoro-alkylidene group of formula - **-CₘHₚF₂ₙ₋ₚ-** with n in the range 1 - 6 and **p** in the range 0-4; where the divalent radical **X** is carbonyl **(-CO-);** and where the monovalent radical **Y** is selected from the group hydrogen **(-H),** bromine **(-Br),** alkyl of formula **-CₘH₂ₘ₊₁** with **m** in the range **1** - 4, or perfluoroalkyl of formula-**CₘF₂ₘ₊₁** with **m** in the range 1 -4, or polyfluoroalkyl of formula **-CₘHₖF₂ₘ₊₁₋ₖ** with **m** in the range 1-4 and **k** in the range 1-2m; the agent including nothing else having any significant environmental impact and which has an atmospheric lifetime longer than 30 days.

4. An agent according to claim 1, a method according to claim 2 or a system according to claim 3, in which the radicals R and Y are linked (by a C-C bond) such as to form a 4-, 5- or 6- membered ring.

5. An agent according to claim 1 or 4, a method according to claim 2 or 4 or a system according to claim 3 or 4, in which the dispersing means (6) comprises means for producing a mist of the chemical substance and entraining the mist in the inert gas.

6. An agent according to claim 1, 4 or 5, a method according to claim 2,4 or 5 or a system according to claim 3,4 or 5, in which the chemical substance is dispersed as a vapour in the inert gas.

7. An agent according to any one of claims 1,4,5 and 6, a method according to any one of claims 2,4,5 and 6 or a system according to any one of claims 3 to 6, in which the groups **Z,X** and **Y** are so selected that the total number of bromine atoms in the molecule does not exceed one.

8. An agent according to any one of claims 1 and 4 to 7, a method according to any one of claims 2 and 4 to 7, or a system according to any one of claims 3 to 7, in which the groups **R** and **Y** are selected such that **n** + **m** lies in the range 1- 6, and **n** - **m** is at least 1.

9. An agent according to any one of claims **1** and 4 to 8, a method according to any one of claims 2 and 4 to 8, or a system according to any one of claims 3 to 8, in which the groups **R,X** and **Y** are chosen so that the total number of carbon atoms in the molecule is in the range 3 - 8.

10. An agent according to claim 9, a method according to claim 9, or a system according to claim 9, in which the total number of the said carbon atoms is in the range 3 - 6.

11. An agent according to any one of claims 1 and 4 to 10, a method according to any one of claims 2 and 4 to 10, or a system according to any one of claims 3 to 10, in which the molecular weight of the molecule lies in the range 150 - 400.

12. An agent according to claim 11, a method according to claim 11, or a system according to claim 11, in which the said molecular weight lies in the range 150 - 350.

13. An agent according to any one of claims 1 and 4 to 12, a method according to any one of claims 2 and 4 to 12, or a system according to any one of claims 3 to 12, in which the groups **R,X** and **Y** are chosen so that the weight% of halogen (fluorine and bromine) in the molecule lies in the range 70 - 90%.

14. An agent according to claim 13, a method according to claim 13, or a system according to claim 13, which the weight% of halogen (fluorine and bromine) in the molecule lies in the range 70-80%.

15. An agent according to any one of claims 1 and 4 to 14, a method according to any one of claims 2 and 4 to 14, or a system according to any one of claims 3 to 14, in which the chemical substance is dodecafluoro-2-methylpentan-3-one.

16. An agent according to any one of claims 1 and 4 to 15, a method according to any one of claims 2 and 4 to 15, or a system according to any one of claims 3 to 15, in which the inert gas comprises one or more of argon, helium, neon, nitrogen and carbon dioxide.

## Patentansprüche

1. Mittel zum Feuerlöschen oder zur Explosionsunterdrückung mit zwei unterdrückenden Teilen, von denen einer eine explosionsunterdrückende chemische Substanz umfasst, die bei normalen Temperaturen und Drücken im Wesentlichen flüssig ist, und der andere ein feuerlöschendes oder explosionsunterdrückendes Inertgas umfasst; wobei die chemische Substanz als eine Suspension in dem Inertgas dispergiert wird, wobei die chemische Substanz bei einer solchen Dispersion mit einer kurzen Atmosphärenverweildauer von weniger als 30 Tagen geringe Auswirkungen auf die Umwelt aufweist; wobei die chemische Substanz ein oder mehrere Chemikalien mit der Struktur **Z-R-X-Y** aufweist, wobei das einwertige Radikal **Z ein** Halogenatom ist, das aus der Gruppe Fluor **(-F)** oder Brom **(-Br)** entnommen wurde; wobei das zweiwertige Radikal **R** eine Perfluor- oder Polyfluoralkylidengruppe der Formel **-CₙHₚF₂ₙ₋ₚ** ist, wobei **n** im Bereich von 1-6 liegt und **p** im Bereich von 0-4 liegt; wobei das zweiwertige Radikal **X** Carbonyl **(-CO-)** ist; und wobei das einwertige Radikal **Y** ausgewählt ist aus der Gruppe Wasserstoff **(-H),** Brom **(-Br),** Alkyl der Formel **-CₘH₂ₘ₊₁**, wobei **m** im Bereich von 1-4 liegt, oder Perfluoralkyl der Formel -**CₘF₂ₘ₊₁**, wobei **m** im Bereich 1-4 liegt, oder Polyfluoralkyl der Formel **-CₘHₖF₂ₘ₊₁₋ₖ**, wobei **m** im Bereich 1-4 und **k** im Bereich 1-2 m liegt; wobei das Mittel nichts anderes mit wesentlichen Umweltauswirkungen und einer Atmosphärenverweildauer von mehr als 30 Tagen enthält.

2. Verfahren zum Feuerlöschen oder zur Explosionsunterdrückung, wobei eine feuerlöschende oder explosionsunterdrückende chemische Substanz, die bei normalen Temperaturen und Drücken in flüssiger oder im Wesentlichen flüssiger Form vorliegt, als eine Suspension in einem feuerlöschenden oder explosionsunterdrückenden Inertgas dispergiert und mit dem Gas in einen zu schützenden Bereich abgegeben wird, wobei die chemische Substanz als eine Suspension im Inertgas dispergiert wird, wobei die chemische Substanz bei einer solchen Dispersion mit einer kurzen Atmosphärenverweildauer von weniger als 30 Tagen geringe Auswirkungen auf die Umwelt aufweist; wobei die chemische Substanz ein oder mehrere Chemikalien mit der Struktur **Z-R-X-Y** aufweist, wobei das einwertige Radikal **Z** ein Halogenatom ist, das aus der Gruppe Fluor **(-F)** oder Brom **(-Br)** entnommen wurde; wobei das zweiwertige Radikal **R eine** Perfluor- oder Polyfluoralkylidengruppe der Formel **-CₙHpF₂ₙ₋ₚ** ist, wobei **n** im Bereich von 1-6 liegt und **p** im Bereich von 0-4 liegt; wobei das zweiwertige Radikal **X** Carbonyl **(-CO-)** ist; und wobei das einwertige Radikal ausgewählt ist aus der Gruppe Wasserstoff **(-H),** Brom **(-Br),** Alkyl der Formel **-CₘH₂ₘ₊₁,** wobei **m** im Bereich von 1-4 liegt, oder Perfluoralkyl der Formel **-CₘF₂ₘ₊₁,** wobei **m** im Bereich 1-4 liegt, oder Polyfluoralkyl der Formel **-CₘHₖF₂ₘ₊₁₋ₖ,** wobei **m** im Bereich 1-4 und **k** im Bereich 1-2 m liegt; wobei das Mittel nichts anderes mit wesentlichen Umweltauswirkungen und einer Atmosphärenverweildauer von mehr als 30 Tagen enthält.

3. System zum Feuerlöschen oder zur Explosionsunterdrückung, umfassend eine Quelle (30; 5) einer feuerlöschenden oder explosionsunterdrückenden chemischen Substanz, die bei normalen Temperaturen und Drücken in flüssiger oder im Wesentlichen flüssiger Form vorliegt, und eine Quelle (32; 14) eines feuerlöschenden oder explosionsunterdrückenden Inertgases unter Druck, ein Mittel (34; 6) zum Dispergieren der chemischen Substanz als eine Suspension in dem Druckgas und ein Abgabemittel (44; 26, 29) zum Abgeben der derart dispergierten chemischen Substanz und des Druckgases in einen zu schützenden Bereich; wobei die chemische Substanz bei einer solchen Dispersion mit einer kurzen Atmosphärenverweildauer von weniger als 30 Tagen geringe Auswirkungen auf die Umwelt aufweist; wobei die chemische Substanz ein oder mehrere Chemikalien mit der Struktur **Z-R-X-Y** aufweist, wobei das einwertige Radikal **Z ein** Halogenatom ist, das aus der Gruppe Fluor **(-F)** oder Brom **(-Br)** entnommen wurde; wobei das zweiwertige Radikal **R** eine Perfluor- oder Polyfluoralkylidengruppe der Formel **-CₙHₚF₂ₙ₋ₚ** ist, wobei **n** im Bereich von 1-6 liegt und **p** im Bereich von 0-4 liegt; wobei das zweiwertige Radikal **X** Carbonyl **(-CO-)** ist; und wobei das einwertige Radikal ausgewählt ist aus der Gruppe Wasserstoff **(-H),** Brom **(-Br),** Alkyl der Formel **-CₘH₂ₘ₊₁,** wobei **m** im Bereich von 1-4 liegt, oder Perfluoralkyl der Formel -**CₘF₂ₘ₊₁,** wobei **m** im Bereich 1-4 liegt, oder Polyfluoralkyl der Formel **-CₘHₖF₂ₘ₊₁₋ₖ,** wobei **m** im Bereich 1-4 und **k** im Bereich 1-2 m liegt; wobei das Mittel nichts anderes mit wesentlichen Umweltauswirkungen und einer Atmosphärenverweildauer von mehr als 30 Tagen enthält.

4. Mittel nach Anspruch 1, Verfahren nach Anspruch 2 oder System nach Anspruch 3, wobei die Radikale **R** und **Y** (durch eine C-C-Bindung) verbunden sind, um einen 4-, 5- oder 6-teiligen Ring zu bilden.

5. Mittel nach Anspruch 1 oder 4, Verfahren nach Anspruch 2 oder 4 oder System nach Anspruch 3 oder 4, wobei das Dispergiermittel (6) ein Mittel zum Erzeugen eines Nebels der chemischen Substanz und zum Mitführen des Nebels in dem Inertgas umfasst.

6. Mittel nach Anspruch 1, 4 oder 5, Verfahren nach Anspruch 2, 4 oder 5 oder System nach Anspruch 3, 4 oder 5, wobei die chemische Substanz als ein Dampf in dem Inertgas dispergiert wird.

7. Mittel nach Anspruch 1, 4, 5 oder 6, Verfahren nach Anspruch 2, 4, 5 oder 6 oder System nach einem der Ansprüche 3 bis 6, wobei die Gruppen **Z, X** und **Y** derart ausgewählt werden, dass die Gesamtanzahl von Bromatomen in dem Molekül nicht höher als eins ist.

8. Mittel nach Anspruch 1 und 4 bis 7, Verfahren nach Anspruch 2 und 4 bis 7 oder System nach einem der Ansprüche 3 bis 7, wobei die Gruppen **R** und **Y** derart ausgewählt werden, dass **n** + **m** im Bereich 1- 6 liegt und **n - m** wenigstens 1 ist.

9. Mittel nach Anspruch 1 und 4 bis 8, Verfahren nach Anspruch 2 und 4 bis 8 oder System nach einem der Ansprüche 3 bis 8, wobei die Gruppen **R, X** und **Y** derart ausgewählt werden, dass die Anzahl der Kohlenstoffatome im Molekül im Bereich 3 - 8 liegt.

10. Mittel nach Anspruch 9, Verfahren nach Anspruch 9 oder System nach Anspruch 9, wobei die Gesamtanzahl der Kohlenstoffatome im Bereich 3 - 6 liegt.

11. Mittel nach Anspruch 1 und 4 bis 10, Verfahren nach Anspruch 2 und 4 bis 10 oder System nach einem der Ansprüche 3 bis 10, wobei das Molekulargewicht des Moleküls im Bereich 150 - 400 liegt.

12. Mittel nach Anspruch 11, Verfahren nach Anspruch 11 oder System nach Anspruch 11, wobei das Molekulargewicht im Bereich 150 - 350 liegt.

13. Mittel nach Anspruch 1 und 4 bis 12, Verfahren nach Anspruch 2 und 4 bis 12 oder System nach einem der Ansprüche 3 bis 12, wobei die Gruppen **R, X** und **Y** derart ausgewählt werden, dass der Gewichtsanteil an Halogen (Fluor und Brom) im Molekül im Bereich 70 - 90 % liegt.

14. Mittel nach Anspruch 13, Verfahren nach Anspruch 13 oder System nach Anspruch 13, wobei der Gewichtsanteil an Halogen (Fluor und Brom) im Molekül im Bereich 70 - 80 % liegt.

15. Mittel nach Anspruch 1 und 4 bis 14, Verfahren nach Anspruch 2 und 4 bis 14 oder System nach einem der Ansprüche 3 bis 14, wobei die chemische Substanz Dodecafluor-2-methylpentan-3-on ist.

16. Mittel nach Anspruch 1 und 4 bis 15, Verfahren nach Anspruch 2 und 4 bis 15 oder System nach einem der Ansprüche 3 bis 15, wobei das Inertgas eins oder mehr von Argon, Helium, Neon, Stickstoff und Kohlendioxid umfasst.

## Revendications

1. Agent de suppression d'incendie ou d'explosion, comportant deux parts actives, une comprenant une substance chimique de suppression d'explosion qui est sensiblement liquide à des températures et sous des pressions normales, et l'autre comprenant un gaz inerte de suppression d'incendie ou d'explosion ; la substance chimique étant dispersée sous forme de suspension dans le gaz inerte, la substance chimique ayant un faible impact sur l'environnement quand on la disperse ainsi, avec une brève durée de vie dans l'atmosphère inférieure à 30 jours ; la substance chimique contenant au moins un produit chimique de structure Z-R-X-Y, le radical monovalent Z étant un atome d'halogène issu du groupe constitué par le fluor (-F) ou le brome (-Br) ; le radical divalent R étant un groupe perfluoro- ou polyfluoro-alkylidène de formule -CₙHₚF₂ₙ₋ₚ, où n est compris entre 1 et 6, et où p est compris entre 1 et 4 ; le radical divalent X étant le carbonyle (-CO-) ; et le radical monovalent Y étant choisi dans l'ensemble constitué de l'hydrogène (-H), du brome (-Br), d'un alkyle de formule -CₘH₂ₘ₊₁ où m est compris entre 1 et 4 ou perfluoroalkyle de formule -CₘF₂ₘ₊₁ où m est compris entre 1 et 4, ou polyfluoroalkyle de formule -CₘHₖF₂ₘ₊₁₋ₖ où m est compris entre 1 et 4 et où k est compris entre 1 et 2m ; l'agent ne comprenant rien d'autre qui ait un impact important sur l'environnement et qui présente une durée de vie dans l'atmosphère supérieure à 30 jours.

2. Procédé de suppression d'incendie ou d'explosion, dans lequel une substance chimique de suppression d'incendie ou d'explosion qui est sous forme liquide ou sensiblement ainsi à des températures et sous des pressions normales est dispersée sous forme de suspension dans un gaz inerte de suppression d'incendie ou d'explosion et évacuée avec le gaz dans une zone à protéger ; la substance chimique étant dispersée sous forme de suspension dans le gaz inerte, la substance chimique ayant un faible impact sur l'environnement quand on la disperse ainsi, avec une brève durée de vie dans l'atmosphère inférieure à 30 jours ; la substance chimique contenant au moins un produit chimique de structure Z-R-X-Y, le radical monovalent Z étant un atome d'halogène issu du groupe constitué par le fluor (-F) ou le brome (-Br) ; le radical divalent R étant un groupe perfluoro- ou polyfluoro-alkylidène de formule -CₙHₚF₂ₙ₋ₚ, où n est compris entre 1 et 6, et où p est compris entre 1 et 4 ; le radical divalent X étant le carbonyle (-CO-) ; et le radical monovalent Y étant choisi dans l'ensemble constitué de l'hydrogène (-H), du brome (-Br), d'un alkyle de formule -CₘH₂ₘ₊₁ où m est compris entre 1 et 4 ou perfluoroalkyle de formule -CₘF₂ₘ₊₁ où m est compris entre 1 et 4, ou polyfluoroalkyle de formule -CₘHₖF₂ₘ₊₁₋ₖ où m est compris entre 1 et 4 et où k est compris entre 1 et 2m ; l'agent ne comprenant rien d'autre qui ait un impact important sur l'environnement et qui présente une durée de vie dans l'atmosphère supérieure à 30 jours.

3. Système de suppression d'incendie ou d'explosion, comprenant une source (30 ; 5) d'une substance chimique de suppression d'incendie ou d'explosion qui est sous forme liquide ou sensiblement ainsi à des températures et sous des pressions normales, et une source (32 ; 14) d'un gaz inerte de suppression d'incendie ou d'explosion sous pression, un moyen (34 ; 6) servant à disperser la substance chimique sous forme de suspension dans le gaz sous pression, et un moyen d'évacuation (44 ; 26, 29) servant à évacuer la substance chimique ainsi dispersée et le gaz sous pression dans une zone à protéger ; la substance chimique étant dispersée sous forme de suspension dans le gaz inerte, la substance chimique ayant un faible impact sur l'environnement quand on la disperse ainsi, avec une brève durée de vie dans l'atmosphère inférieure à 30 jours ; la substance chimique contenant au moins un produit chimique de structure Z-R-X-Y, le radical monovalent Z étant un atome d'halogène issu du groupe constitué par le fluor (-F) ou le brome (-Br) ; le radical divalent R étant un groupe perfluoro- ou polyfluoro-alkylidène de formule -CₙHₚF₂ₙ₋ₚ, où n est compris entre 1 et 6, et où p est compris entre 0 et 4 ; le radical divalent X étant le carbonyle (-CO-) ; et le radical monovalent Y étant choisi dans l'ensemble constitué de l'hydrogène (-H), du brome (-Br), d'un alkyle de formule - CₘH₂ₘ₊₁ où m est compris entre 1 et 4 ou perfluoroalkyle de formule -CₘF₂ₘ₊₁ où m est compris entre 1 et 4, ou polyfluoroalkyle de formule -CₘHₖF₂ₘ₊₁₋ₖ où m est compris entre 1 et 4 et où k est compris entre 1 et 2m ; l'agent ne comprenant rien d'autre qui ait un impact important sur l'environnement et qui présente une durée de vie dans l'atmosphère supérieure à 30 jours.

4. Agent selon la revendication 1, procédé selon la revendication 2 ou système selon la revendication 3, dans lequel les radicaux R et Y sont liés (par une liaison C-C) de manière à former un cycle à 4, 5 ou 6 membres.

5. Agent selon la revendication 1 ou 4, procédé selon la revendication 2 ou 4 ou système selon la revendication 3 ou 4, dans lequel le moyen de dispersion (6) comprend un moyen servant à produire un brouillard de la substance chimique et à entraîner le brouillard dans le gaz inerte.

6. Agent selon la revendication 1, 4 ou 5, procédé selon la revendication 2, 4 ou 5 ou système selon la revendication 3, 4 ou 5, dans lequel la substance chimique est dispersée sous forme de vapeur dans le gaz inerte.

7. Agent selon l'une quelconque des revendications 1, 4, 5 et 6, procédé selon l'une quelconque des revendications 2, 4, 5 et 6 ou système selon l'une quelconque des revendications 3 à 6, dans lequel les groupes Z, X et Y sont choisis de sorte que le nombre total d'atomes de brome dans la molécule ne dépasse pas un.

8. Agent selon l'une quelconque des revendications 1 et 4 à 7, procédé selon l'une quelconque des revendications 2 et 4 à 7 ou système selon l'une quelconque des revendications 3 à 7, dans lequel les groupes R et Y sont choisis de sorte que n + m soit compris entre 1 et 6 et que n - m soit supérieur ou égal à 1.

9. Agent selon l'une quelconque des revendications 1 et 4 à 8, procédé selon l'une quelconque des revendications 2 et 4 à 8 ou système selon l'une quelconque des revendications 3 à 8, dans lequel les groupes R, X et Y sont choisis de sorte que le nombre total d'atomes de carbone présent dans la molécule soit compris entre 3 et 8.

10. Agent selon la revendication 9, procédé selon la revendication 9 ou système selon la revendication 9, dans lequel le nombre total desdits atomes de carbone est compris entre 3 et 6.

11. Agent selon l'une quelconque des revendications 1 et 4 à 10, procédé selon l'une quelconque des revendications 2 et 4 à 10 ou système selon l'une quelconque des revendications 3 à 10, dans lequel la masse moléculaire de la molécule est comprise entre 150 et 400.

12. Agent selon la revendication 11, procédé selon la revendication 11 ou système selon la revendication 11, dans lequel ladite masse moléculaire de la molécule est comprise entre 150 et 350.

13. Agent selon l'une quelconque des revendications 1 et 4 à 12, procédé selon l'une quelconque des revendications 2 et 4 à 12 ou système selon l'une quelconque des revendications 3 à 12, dans lequel les groupes R, X et Y sont choisis de sorte que le pourcentage en poids d'halogène (fluor et brome) dans la molécule soit compris entre 70 et 90 %.

14. Agent selon la revendication 13, procédé selon la revendication 13 ou système selon la revendication 13, dans lequel le pourcentage en poids d'halogène (fluor ou brome) dans la molécule est compris entre 70 et 80 %.

15. Agent selon l'une quelconque des revendications 1 et 4 à 14, procédé selon l'une quelconque des revendications 2 et 4 à 14 ou système selon l'une quelconque des revendications 3 à 14, dans lequel la substance chimique est la dodécafluoro-2-méthylpentan-3-one.

16. Agent selon l'une quelconque des revendications 1 et 4 à 15, procédé selon l'une quelconque des revendications 2 et 4 à 15 ou système selon l'une quelconque des revendications 3 à 15, dans lequel le gaz inerte contient au moins soit de l'argon, soit de l'hélium, soit du néon, soit de l'azote, soit du dioxyde de carbone.
